# EUROPÄISCHE PATENTANMELDUNG

(11) **EP 1 389 765 A2**
(43) Veröffentlichungstag der Anmeldung: **18.02.2004**
(21) Anmeldenummer: 03017942.8
(22) Anmeldetag: 06.08.2003
(51) Int. Cl.: G06F 17/30

(54) **Computersystem und Verfahren zur Abfrage von Daten aus einer Menge von unabhängigen Datenbanken**

(30) Priorität: 13.08.2002 DE 10237369
(71) Anmelder: CompuGroup Holding AG, 56070 Koblenz (DE)
(72) Erfinder: Broer, Jan, 56072 Koblenz (DE)
(74) Vertreter: Richardt, Markus Albert

(57) **Zusammenfassung**

Die Erfindung betrifft ein Verfahren zur Abfrage von Daten aus einer Menge von unabhängigen Datenbanken zur Vorbereitung einer Datenerhebung, wobei jede der Datenbanken Instanzen einer Entität aufweist, mit folgenden Schritten:
- Übertragung eines Suchprofils und einer Mindestanzahl an die Datenbanken der Menge von unabhängigen Datenbanken,
- für jede der Datenbanken:
   a) Ermittlung der Anzahl der Instanzen der Datenbank, für die das Suchprofil zutrifft,
   b) wenn die Anzahl die Mindestanzahl erreicht: Ausgabe eines Signals.

## Beschreibung

Die Erfindung betrifft ein Verfahren zur Abfrage von Daten aus einer Menge von unabhängigen Datenbanken, insbesondere von Patientendatenbanken für die Zwecke einer Anwendungsbeobachtung, sowie ein entsprechendes Computerprogrammprodukt und Computersystem.

Eine Anwendungsbeobachtung (AWB) ist eine Beobachtungsstudie, die dazu bestimmt ist, Erkenntnisse bei der Anwendung zugelassener, registrierter oder fiktiv zugelassener Arzneimittel zu sammeln. Ihr besonderes Charakteristikum ist die weitestgehende Nichtbeeinflussung des individuellen Arzt-Patienten-Verhältnisses in Bezug auf Indikationsstellung sowie Wahl und Durchführung der Therapie. Eine AWB kann ohne Vergleichsgruppe, d.h. arzneimittelspezifisch, oder mit zwei oder mehr zu vergleichenden Gruppen, d.h. insbesondere indikationsorientiert, angelegt sein. Da eine AWB Erkenntnisse über die Anwendung von Arzneimitteln unter Routinebedingungen liefern sollen, wird sie in der Regel mit Handelsware durchgeführt.

AWB erfordern eine Planung, Durchführung, Aus- und Bewertung nach dem Stand der wissenschaftlichen Erkenntnis der beteiligten Disziplinen. Sie müssen eine Zielsetzung verfolgen, die als detaillierte Fragestellung vorab formuliert sein muß. Das gewählte Design (Basis eines Vergleichs, zeitlicher Umfang und Untersuchungsumfang beim einzelnen Patienten, Patientenzahl) und die geplanten Methoden (Datenerhebung und Auswertung) müssen zur Beantwortung dieser Frage geeignet sein. Eine AWB ist in der Regel prospektiv, ggf. mit zurückverlegtem Anfangspunkt, durchzuführen und orientiert sich in Anlage und Durchführung an einer Kohortenstudie. Sie kann auch auf geeigneten pharmakoepidemiologischen Datenbeständen basieren.

AWB sind eines von mehreren methodischen Instrumenten, um Erkenntnisse zu auf dem Markt verfügbaren Arzneimitteln zu gewinnen. Andere wichtige Instrumente sind Klinische Prüfungen der Phase IV sowie Fall-Kontroll-Studien, Querschnittstudien, Korrelationsstudien mit aggregierten Daten, Auswertungen von Registern und Spontan-Meldesysteme.

Mögliche Ziele von AWB sind:
- das Gewinnen von Erkenntnissen zur Arzneimittel-Utilisation (Verordnungsverhalten und Verschreibungsgewohnheiten, Beachtung der Fach- und Gebrauchsinformationen, Akzeptanz und Compliance, Praktikabilität, Beachtung von Zulassungsauflagen etc.);
- das Gewinnen von Erkenntnissen über direkte, indirekte und intangible Kosten, die mit der routinemäßigen Anwendung einer Therapie entstehen bzw. in Zusammenhang stehen;
- das Vertiefen von Erkenntnissen zu bekannten unerwünschten Arzneimittelwirkungen (UAW) unter routinemäßiger Anwendung (Überprüfung der zu erwartenden UAW, Häufigkeitsabschätzungen);
- das Gewinnen von Erkenntnissen zu bisher unbekannten, insbesondere seltenen UAW sowie zu Wechselwirkungen;
- das Erweitern von Erkenntnissen zur Wirksamkeit (z.B. unter Bedingungen der routinemäßigen Anwendung; in Gruppen, die in klinische Prüfungen nicht eingeschlossen wurden, in Subgruppen; zur Charakterisierung von Non-respondern; etc.).

Vor Beginn einer AWB wir ein Studienplan erstellt, der dem aktuellen Stand der medizinischen und biometrischen Wissenschaft entspricht. Seine wesentlichen Bestandteile sind der Beobachtungs- sowie der Auswertungsplan.

Der Studienplan soll folgende Angaben enthalten:
- Formulierung einer (oder mehrerer) detaillierten(r) Fragestellung(en) sowie eine Begründung, daß die AWB für ihre Beantwortung das geeignete Instrument ist;
- Beschreibung des Patientenzugangs und ggf. des Vorgehens zur Auswahl der beteiligten Ärzte (Zentren);
- Definition der einzubeziehenden Patienten sowie gegebenenfalls Beschreibung des Vorgehens für den Patienteneinschluß;
- Beschreibung der Maßnahmen zum Erreichen von Repräsentativität (für Ärzte und Patienten)
- Festlegung der zu erhebenden Merkmale, eine Beschreibung ihrer Relevanz sowie ihrer Stellung für die Beantwortung der Fragestellung (Zielgröße, Einflußgröße, Störgröße);
- Diskussion möglicher Störgrößen und Beschreibung von Maßnahmen zu ihrer Kontrolle
- Zeitraster der Beobachtung;
- Beschreibung der für die Beobachtung benötigten Erhebungsinstrumente inkl. der Begründung, daß die hiermit erhobenen Daten geeignet sind, die formulierte Fragestellung zu beantworten;
- Begründung der Zahl einzubeziehender Patienten;
- Beschreibung von Art und Umfang der Dokumentation;
- Regelung der Berichtswege zu UAW;
- Beschreibung von Maßnahmen zur Qualitätssicherung;
- Beschreibung der statistischen Auswertung;
- Regelung der Verantwortlichkeiten (Sponsor, Studienleiter, verantwortlicher Biometriker etc.);
- Regelungen für Berichterstellung inkl. biometrischer und medizinischer Bewertung.

Weiteres hierzu findet sich in den "Empfehlungen zur Durchführung von Anwendungsbeobachtungen", Deutsche Gesellschaft für Medizinische Informatik, Biometrie und Epidemiologie (GMDS), (http://www.gmds.de/texte/onlinedocs/empfehlungen/empf_anwendungsbeobac htungen.html).

Aus dem Stand der Technik ist es bereits bekannt, Anwendungsbeobachtungen online durchzuführen. Die Erhebungsdaten werden dabei von den Prüfärzten online über elektronische Formulare erfasst und zentral auf einem Web-Server gespeichert. Dies hat den Vorteil, dass die Studiendaten sofort verfügbar und jederzeit auswertbar sind. Ein entsprechendes Softwareprodukt ist von der Firma DATAPHARM Netsystems AG, Geschäftsbereich DATAPHARM New Media, 82110 Germering, http://www.datapharmnewmedia.de/produkte/onlineawbs.htm erhältlich.

Die Datenerfassung zur Anwendungsbeobachtung lässt sich besonders bequem über das Internet durchführen. Der Arzt dokumentiert über einen Zeitraum von zum Beispiel zwölf Wochen Daten, die routinemäßig bei der Behandlung eines Patienten erfasst werden. Nach Abschluss aller Dokumentationen werden die Ergebnisse ausgewertet und allen Teilnehmern zur Verfügung gestellt (vgl. "Aventis Pharma starten Online-Anwendungsbeobachtung mit dem Insulin-FertigPen Lantus®OptiSet®", http://www.diabsite.de/aktuelles/nachrichten/2001/011027.html).

Aus der US 6,205,455 ist ferner ein Dokumentationssystem bekannt, welches die Erstellung einer Dokumentation für ein Arzneimittel-Genehmigungsverfahren unterstützt.

Ein gemeinsamer Nachteil von vorbekannten Programmen für die Online-Anwendungsbeobachtung oder die Online-Erfassung von anderen medizinischen, Kundendaten oder sonstigen Daten, ist, dass zunächst die an einer solchen Datenerhebung teilnehmenden Entitäten zu bestimmen sind.

Bei einer Anwendungsbeobachtung handelt es sich hierbei typischerweise um Arztpraxen oder andere medizinische Einrichtungen, die jeweils mit voneinander unabhängigen Praxis-EDV-Systemen und entsprechenden Patientendatenbanken ausgerüstet sind. Bevor eine Datenerfassung online durchgeführt werden kann, ist es also erforderlich, aus den Arztpraxen oder anderen medizinischen Einrichtungen diejenigen zu ermitteln, welche an der Studie teilnehmen sollen.

Dies erfolgt im Stand der Technik im Allgemeinen so, dass beispielsweise Pharmavertreter bei einem Besuch persönlich anfragen, ob der betreffende Arzt für die Teilnahme an einer Anwendungsbeobachtung bereit ist. Bei dieser Anfrage ist zunächst nicht klar, ob die Arztpraxis überhaupt eine ausreichende Anzahl von Patienten hat, die sie für die Teilnahme an der Anwendungsbeobachtung qualifiziert.

Selbst wenn der Arzt also der Teilnahme zustimmt, kann es sich im Nachhinein herausstellen, dass diese Arztpraxis für die Teilnahme an der Anwendungsbeobachtung nicht geeignet ist. Dieses aus dem Stand der Technik bekannte Verfahren zur Ermittlung von Arztpraxen bzw. von entsprechenden Patientendatenbanken der Arztpraxen für die Durchführung von Anwendungsbeobachtungen ist also in höchstem Maße ineffizient.

Daneben ist es ferner aus dem Stand der Technik bekannt im Internet eine Aufforderung für die Teilnahme an einer Anwendungsbeobachtung zu veröffentlichen. Auch hierbei ist nachteilig, dass diese Aufforderung unspezifisch ist, das heißt, dass sich gleichermaßen geeignete wie auch ungeeignete Arztpraxen daraufhin melden können. Erst im Nachhinein kann dann festgestellt werden, ob eine Arztpraxis, die zur Teilnahme an der Anwendungsbeobachtung bereit ist, hierfür auch geeignet ist.

Der Erfindung liegt daher die Aufgabe zu Grunde ein verbessertes Verfahren zur Ermittlung von Datenbank aus einer Menge von unabhängigen Datenbanken zu schaffen, sowie ein entsprechendes Computerprogrammprodukt und ein Computersystem.

Die der Erfindung zu Grunde liegenden Aufgaben werden jeweils mit den Merkmalen der unabhängigen Patentansprüche gelöst. Bevorzugte Ausführungsformen der Erfindung sind in den abhängigen Patentansprüchen angegeben.

Die Erfindung ermöglicht eine effiziente und automatisierte Abfrage von Daten aus einer Menge von unabhängigen Datenbanken, die je für sich nicht durch einen Dritten zugänglich sind. Bei den Datenbanken kann es sich zum Beispiel jeweils um Patientendatenbanken von Arztpraxen oder anderen medizinischen Einrichtungen handeln, die unabhängig voneinander betrieben werden und auf die durch einen Dritten insbesondere aus Gründen des Datenschutzes nicht zugegriffen werden kann.

Nach einer bevorzugten Ausführungsform der Erfindung erfolgt eine automatisierte Auswahl von Datenbanken aus einer Menge von unabhängigen Datenbanken, indem ein Suchprofil an die Datenbanken verteilt wird, sowie eine Angabe hinsichtlich einer Mindestanzahl von Instanzen. Wenn eine der Datenbanken Instanzen aufweist, die dem Suchprofil entsprechen, und die Anzahl der Instanzen die Mindestanzahl erreicht oder überschreitet, so qualifiziert sich diese Datenbank für die Teilnahme an der Datenerfassung und gibt ein entsprechendes Signal ab.

Nach einer bevorzugten Ausführungsform der Erfindung kann ein Benutzer dieses Signal bestätigen und hiermit seine Zustimmung zur Teilnahme an der Datenerhebung zum Ausdruck bringen. Vorzugsweise wird die Bestätigung an einen Zentralcomputer übertragen. Alternativ wird die Zustimmung des Benutzers an der Teilnahme der Datenerhebung auf herkömmlichem Wege wie beispielsweise per E-Mail, Fax oder per Post übertragen.

Nach einer bevorzugten Ausführungsform der Erfindung erfolgt zunächst eine Definition der in einer Anwendungsbeobachtung einzubeziehenden Patienten. Mit Hilfe der Felder der elektronischen Patientenkartei wird dann ein entsprechendes Suchprofil formuliert. Die Formulierung des Suchprofils kann dabei zum Beispiel durch geeignete Verknüpfung von Boolschen Operatoren, die auf Felder der elektronischen Patientenkartei angewendet werden, erfolgen.

Da eine Teilnahme einer Arztpraxis oder einer anderen medizinischen Einrichtung an einer Anwendungsbeobachtung nur sinnvoll ist, wenn eine Mindestanzahl von relevanten Patienten vorhanden ist, wird ferner eine solche Mindestanzahl festgelegt. Vorzugsweise wird dann das Suchprofil und die Mindestanzahl an die verschiedenen Datenbanken, das heißt an die Arztpraxen oder anderen medizinischen Einrichtungen, übertragen. Die Übertragung kann online erfolgen oder auch im Rahmen eines Software-Updates der Praxissoftware. Im letzteren Fall werden die entsprechenden Daten zusammen mit dem Software-Update zum Beispiel auf einer CD-Rom gespeichert und anlässlich des Software-Updates mit in die Praxissoftware eingelesen.

Nach dem Einlesen des Suchprofils in die Praxissoftware wird das Suchprofil gestartet und auf die jeweilige Datenbank angewendet. Wenn sich die Mindestanzahl von "Treffern" ergibt, wird beispielsweise ein Selbstanzeigeformular generiert. Das Selbstanzeigeformular beinhaltet vorzugsweise eine vorgefertigte Erklärung, die der betreffende Arzt zum Ausdruck seines Einverständnisses an der Teilnahme an der Anwendungsbeobachtung abgeben kann.

Vorzugsweise wird das Selbstanzeigeformular mit einer Kennung oder Adresse der Arztpraxis und / oder einer Adresse der betreffenden Datenbank generiert. Beispielsweise kann das Selbstanzeigeformular in Form einer E-Mail erzeugt werden; zur Abgabe der Teilnahmeerklärung muss der Arzt dann nur noch auf "Senden" klicken. Alternativ wird beispielsweise ein Word-Dokument erzeugt, welches ausgedruckt, unterschrieben und dann zum Beispiel an das Pharmaunternehmen, welches die Anwendungsbeobachtung durchführt, gefaxt werden kann.

Nach einer weiteren bevorzugten Ausführungsform der Erfindung werden die mit den Selbstanzeigeformularen übermittelten Adressen auf einem Zentral-Server Computer für die Zwecke der späteren Durchführung der Anwendungsbeobachtung gespeichert.

Nach einer weiteren bevorzugten Ausführungsform der Erfindung erfolgt keine Selbstanzeige gegenüber einer Zentralinstanz, sondern der Betreiber der jeweiligen Datenbank erhält eine Liste derjenigen Instanzen seiner Datenbank, für die das Suchprofil zutrifft. Die Mindestanzahl kann dabei im Prinzip beliebig gewählt werden und insbesondere auch "1" betragen. Ein Anwendungsfall hierfür ist gegeben, wenn eine Krankenkasse nach Patienten für die Teilnahme an einem sogenannten Disease-Management-Program sucht. Mit dem Suchprofil wird dann die entsprechende Patientengruppe definiert. Wenn das Suchprofil auf der Praxis-EDV einer Arztpraxis gestartet wird, so erhält der Arzt gegebenenfalls eine Liste mit einem oder mehreren seiner Patienten, die den Kriterien des Suchprofils entsprechen. Diese Liste kann der Arzt zum Beispiel ausdrucken und die betreffenden Patienten bei einem nächsten Arzttermin über die Möglichkeit der Teilnahme an dem Disease-Management-Program informieren. Alternativ können auch zum Beispiel automatisch durch die Praxis-EDV Informationsschreiben an die betroffenen Patienten generiert werden, um die Patienten über diese Möglichkeit zu informieren. Diese Patienten können sich dann direkt oder über ihren Arzt bei der betreffenden Krankenkasse melden, um gegebenenfalls ihren Wunsch zur Teilnahme an dem angebotenen Disease-Management-Program mitzuteilen.

Die eigentliche Anwendungsbeobachtung kann dann online oder auch offline durchgeführt werden.

Im weiteren werden bevorzugte Ausführungsbeispiele der Erfindung mit Bezugnahme auf die Zeichnungen näher erläutert. Es zeigen:
- Figur 1: ein Flussdiagramm einer ersten Ausführungsform der Erfindung,
- Figur 2: ein Blockdiagramm einer Ausführungsform eines Systems für die Auswahl von unabhängigen Datenbanken,
- Figur 3: ein Flussdiagramm einer zweiten bevorzugten Ausführungsform zur Auswahl von Datenbanken für die Durchführung einer Anwendungsbeobachtung.

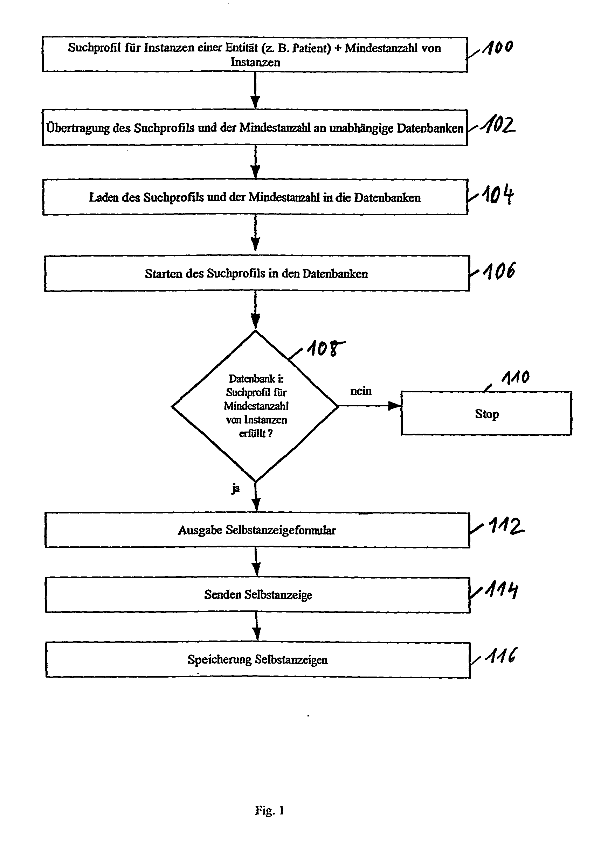

Das Verfahren der Figur 1 bezieht sich auf eine Menge von unabhängigen Datenbanken, die je für sich zum Beispiel von einer Arztpraxis, einer anderen medizinischen Einrichtung oder von einer Einrichtung auf nicht medizinischem Gebiet betrieben werden. Jede der Datenbanken dient zur Erfassung von Instanzen einer Entität, die zu dem jeweiligen Betreiber der Datenbank gehört.

In dem Fall einer Arztpraxis handelt es sich beispielsweise um die Patienten, die in der Praxis-Datenbank erfasst sind. Wenn man von der Entität "Patient" ausgeht, sind also in den Datenbanken jeweils Instanzen dieser Entität gespeichert, das heißt bestimmte Patientendaten in einer elektronischen Patientenkarteikarte.

Zur Abfrage von Daten aus der Menge der unabhängigen Datenbanken wird in dem Schritt 100 zunächst ein Suchprofil für die Instanzen der Entität festgelegt. Ein Suchprofil kann beispielsweise durch eine Boolsche Verknüpfung von attributiven Eigenschaften der Entität formuliert werden. Ferner wird eine Mindestanzahl von Instanzen, die das Suchprofil erfüllen, festgelegt.

In dem Schritt 102 werden das Suchprofil und die Mindestanzahl an die unabhängigen Datenbanken übertragen. Dabei können verschiedene Übertragungswege verwendet werden: Zum einen kann die Übertragung online von einem Zentral-Server Computer beispielsweise über das Internet an die verschiedenen unabhängigen Datenbanken erfolgen. Alternativ kann die Übertragung auch im Wege eines Software-Updates durchgeführt werden.

Hierzu werden das Suchprofil und die Mindestanzahl auf einem Datenträger, wie zum Beispiel einer CD-Rom, zusammen mit dem Software-Update gespeichert. Anlässlich der Installation des Software-Updates werden auch die Daten betreffend des Suchprofils und der Mindestanzahl in das entsprechende Datenbankprogramm eingespeist.

In dem Schritt 104 erfolgt also das Laden des Suchprofils und der Mindestanzahl in die Datenbanken. Dies kann je nach dem gewählten Übertragungsweg gleichzeitig oder mit einem mehr oder weniger großen zeitlichen Versatz geschehen.

In dem Schritt 106 werden die Suchprofile in den Datenbanken gestartet. Hierzu wird beispielsweise das Boolsche Suchprofil von einer Datenbanksoftware ausgewertet und eine entsprechende Datenbanksuche durchgeführt. Diese Datenbanksuche auf Grund des Suchprofils hat als Resultat keinen oder eine bestimmte Anzahl von "Treffern", das heißt von Instanzen der Entität in der Datenbank, die mit dem Suchprofil übereinstimmen.

In dem Schritt 108 wird die in einer Datenbank i der Datenbanken ermittelte Anzahl von Instanzen mit der Mindestanzahl verglichen. Wenn die Anzahl der Instanzen, die das Suchprofil erfüllen, nicht ausreichend groß ist, so stoppt der Ablauf in dem Schritt 110.

Wenn die Anzahl der Instanzen, die das Suchprofil erfüllen, jedoch ausreichend groß ist, wird in dem Schritt 112 ein Selbstanzeigeformular generiert und ausgegeben. Dieses Formular beinhaltet eine Erklärung, wonach der Betreiber der betreffenden Datenbank i zur Teilnahme an einer bestimmten Datenerhebung bereit ist. Der Text dieser Erklärung kann zum Beispiel zusammen mit dem Suchprofil und der Mindestanzahl übertragen werden.

In dem Schritt 114 wird die Selbstanzeige abgesendet. Dies kann zum Beispiel in Form einer E-Mail oder eines Fax erfolgen.

In dem Schritt 116 wird eine empfangene Selbstanzeige zum Beispiel von einem Zentral-Server Computer gespeichert. In dem Zentral-Server Computer werden die eingehenden Selbstanzeigen gesammelt, wodurch die für die geplante Datenerhebung benötigten Datenbanken aus der Menge der unabhängigen Datenbanken ermittelt werden.

Der Schritt 108 und ggf. die Schritte 110 oder 112 bis 116 werden für jede der Datenbanken unabhängig voneinander durchgeführt.

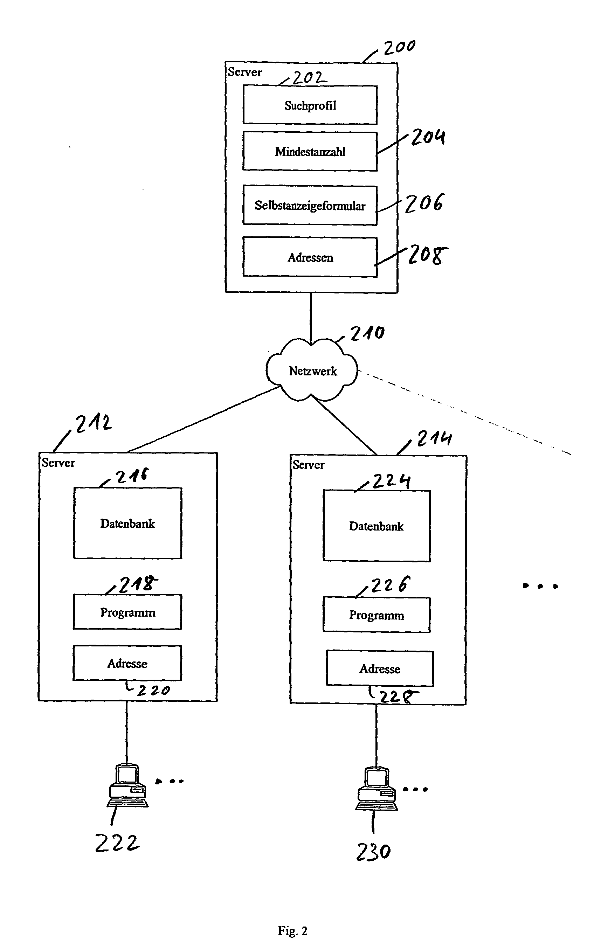

Die Figur 2 zeigt ein Blockdiagramm eines erfindungsgemäßen Systems. Das System hat einen Server Computer 200 beispielsweise eines Pharmaunternehmens oder einer Krankenkasse. In dem Server Computer 200 ist ein Suchprofil 202 sowie eine Mindestanzahl 204 gespeichert. Ferner sind in dem Server Computer 200 Daten 206 zur Generierung eines Selbstanzeigeformulars gespeichert.

Vorzugsweise beinhaltet der Server Computer 200 darüber hinaus eine Datenbank 208 zur Speicherung der Adressen der Datenbanken, für die eine Teilnahme an der Datenerhebung in Frage kommt. Ferner dient die Datenbank 208 zur Speicherung derjenigen Adressen von Datenbanken, die an der Datenerhebung teilnehmen sollen, d.h. für die eine Selbstanzeige vorliegt.

Der Server Computer 200 ist über ein Netzwerk 210, beispielsweise das Internet, mit weiteren Server Computern 212, 214, ... verbindbar. Bei den Server Computern 212, 214, ... handelt es sich beispielsweise um Server Computer von Arztpraxen oder anderen medizinischen Einrichtungen.

Der Server Computer 212 beinhaltet eine Datenbank 216 zur Speicherung von Patientendaten. Zur Speicherung der Patientendaten wird für jeden Patienten eine sogenannte elektronische Patientenkarteikarte angelegt. Diese beinhaltet verschiedene Eingabefelder zur Speicherung der persönlichen Daten des Patienten, von Anamnesedaten und Daten zur Speicherung des Krankheitsund Behandlungsverlaufs sowie zur Medikamentierung.

Ferner hat der Server Computer 212 ein Programm 218, das den Funktionsumfang der üblichen Praxissoftware aufweist (vgl. die Praxissoftwareprodukte Onmed, Dorsymed, Sysmed-KDV und V-doc der CompuMED Praxiscomputer GmbH & Co. KG).

Ferner ist in dem Server Computer 212 eine Adresse 220 gespeichert. Bei der Adresse 220 kann es sich um eine elektronische Adresse, wie zum Beispiel eine Internet-Adresse handeln, die es dem Server Computer 200 erlaubt, den Server Computer 212 bzw. dessen Datenbank 216 zu adressieren. Alternativ oder zusätzlich handelt es sich bei der Adresse 220 um den Namen, Anschrift, Fax- und Telefonnummer sowie gegebenenfalls E-Mail Adresse der betreffenden Arztpraxis.

Mit dem Server Computer 212 sind Arbeitsplatzrechner 222 der Arztpraxis verbunden.

Die Praxis-EDV der Arztpraxis mit dem Server Computer 214 ist prinzipiell gleich aufgebaut: Der Server Computer 214 hat die Datenbank 224, das Programm 226 und die Adresse 228. An den Server Computer 214 sind ein oder mehrere Arbeitsplatzrechner 230 angeschlossen. Prinzipiell gleich sind ebenfalls die Praxis-EDV-Systeme der weiteren über das Netzwerk 210 adressierbaren Arztpraxen aufgebaut.

Zur Durchführung einer Datenerhebung, zum Beispiel für die Zwecke einer Anwendungsbeobachtung, müssen zunächst die an der Datenerhebung teilnehmenden Arztpraxen ausgewählt werden. Dies erfolgt so, dass ein Suchprofil 202 für den relevanten Patiententyp formuliert wird, sowie eine Mindestanzahl 204 solcher relevanter Patienten pro Arztpraxis. Ferner wird der Text für ein Selbstanzeigeformular (Daten 206) erstellt; hierbei kann es sich zum Beispiel um eine Word-Dokumentenvorlage handeln.

Das Suchprofil 202, die Mindestanzahl 204 und die Daten 206 werden dann von dem Server Computer 200 über das Netzwerk 210 an die Server Computer 212, 214, ... verteilt, wobei auf die Adressen 220, 228, ... , die in der Datenbank 208 gespeichert sind, zugegriffen wird.

Das Suchprofil 202 wird dann von dem Programm 218 mit Bezug auf die Datenbank 216 ausgeführt. Die Anzahl der gegebenenfalls erhaltenen "Treffer" in der Datenbank 216 wird dann von dem Programm 218 mit der Mindestanzahl 204 verglichen. Wenn die Anzahl der Treffer die Mindestanzahl 204 erreicht oder übersteigt, greift das Programm 218 auf die auf den Server Computer 212 übertragenen Daten 206 zu und generiert ein Selbstanzeigeformular mit der Adresse 220.

Dieses wird auf einem der Arbeitsplatzrechner 222 angezeigt, so dass es ausgedruckt, unterschrieben und an den Betreiber des Server Computers 200 gefaxt werden kann. Dort wird dann die Adresse 220 zur Identifizierung der so für die Teilnahme an der Datenerhebung ermittelten Datenbank 216 in der Datenbank 208 in einer gesonderten Liste gespeichert.

Alternativ kann auch von dem Programm 218 zum Beispiel eine E-Mail mit dem Selbstanzeigeformular generiert werden. In diesem Fall muss lediglich auf "Senden" geklickt werden, so dass die E-Mail über das Netzwerk 210 an den Server Computer 200 übertragen wird. Auch in diesem Fall wird die Adresse 220 in der Datenbank 208 in der gesonderten Liste abgespeichert. Der Vorteil hierbei ist, dass in dieser Ausführungsform das manuelle Eintragen der Adresse 220 in die Datenbank 208 entfallen kann.

Dieser Ablauf ist für die Server Computer 214, ... und die entsprechenden Praxis-EDV-Systeme prinzipiell gleich. Die einzelnen Datenbankabfragen mit dem Suchprofil 202 werden dabei in den einzelnen Praxis-EDV-Systemen unabhängig voneinander durchgeführt.

Nach einer weiteren Ausführungsform der Erfindung erfolgt die Übertragung des Suchprofils 202, der Mindestanzahl 204 und der Daten 206 nicht online über das Netzwerk 210, sondern im Rahmen der Pflege der Programme 218, 226,... In diesem Fall übergibt der Betreiber des Server Computers 200 dem Hersteller der Programme 218, 226, ... das Suchprofil 202, die Mindestanzahl 204 sowie die Daten 206. Der Hersteller der Programme 218, 226, ... speichert diese Daten zusammen mit einem Software-Update zum Beispiel auf eine CD-Rom. Diese CD-Rom wird dann an die verschiedenen Arztpraxen verteilt und in die Praxis-EDV eingespielt.

Von besonderem Vorteil ist hierbei, dass die Notwendigkeit für ein persönliches Anfragen bei den Arztpraxen, ob eine Bereitschaft zur Teilnahme an der Datenerhebung besteht, entfallen kann. Eine solche Anfrage wird erfindungsgemäß automatisch in Form eines Selbstanzeigeformulars generiert und zwar nur dann, wenn vorab festgestellt worden ist, dass die betreffende Arztpraxis eine Mindestanzahl von relevanten Patienten aufweist. Hierdurch werden online Anwendungsbeobachtungen effizient und mit geringem technischen Aufwand vorbereitet.

Die vorliegende Erfindung ist jedoch nicht auf Client-Server-EDV-Systeme beschränkt; vielmehr können z.B. die Arztpraxen oder die anderen medizinischen Einrichtungen auch an Stelle von Server Computern 212, 214,... und daran angeschlossenen Arbeitsplatzrechnern 222,230,... über Einzelplatzsysteme verfügen.

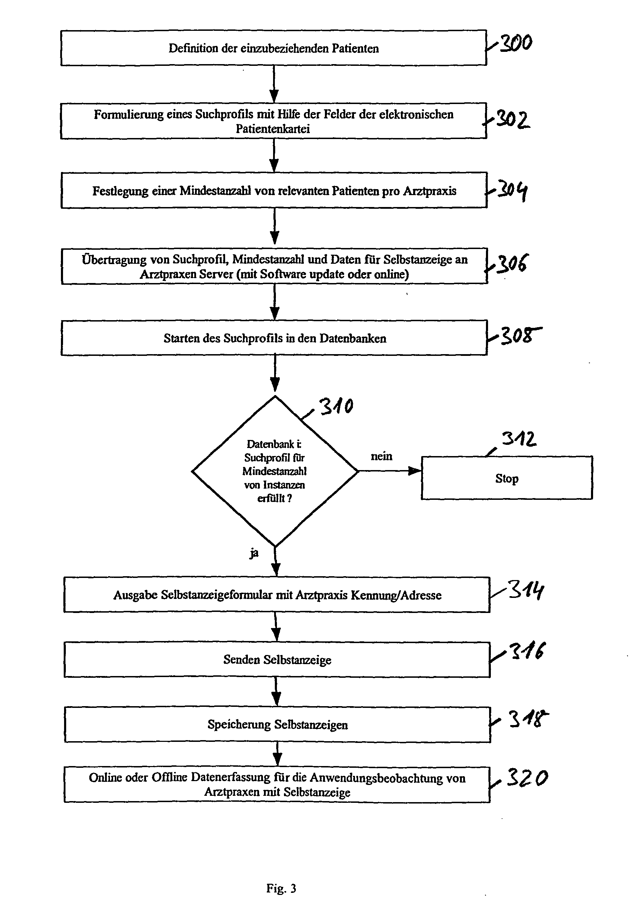

Die Figur 3 zeigt ein entsprechendes Flussdiagramm. In dem Schritt 300 erfolgt eine Definition der in die Anwendungsbeobachtung einzubeziehenden Patienten. In dem Schritt 302 wird ein entsprechendes Suchprofil mit Hilfe der Felder der elektronischen Patientenkartei formuliert. Ferner wird in dem Schritt 304 eine Mindestanzahl von relevanten Patienten pro Datenbank, das heißt pro Arztpraxis festgelegt.

In dem Schritt 306 werden das Suchprofil und die Mindestanzahl an die Arztpraxen-Server übertragen. Dies kann online oder offline erfolgen, letzteres zum Beispiel im Rahmen eines Software-Updates. Vorzugsweise werden hierbei auch Daten zur Generierung eines Selbstanzeigeformulars mit übertragen.

In dem Schritt 308 werden die Suchprofile in den verschiedenen Datenbanken der Arztpraxen gestartet, nachdem zum Beispiel das Software-Update installiert worden ist.

In dem Schritt 310 wird für eine bestimmte Datenbank i der involvierten Patientendatenbanken geprüft, ob das Suchprofil für eine Mindestanzahl von Patienten erfüllt ist. Wenn dies nicht der Fall ist, bricht der Ablauf in dem Schritt 312 ab und es kommt zu keiner Meldung an den Benutzer der Praxis-EDV.

Wird hingegen das Suchprofil für die Mindestanzahl von Patienten erfüllt, so wird in dem Schritt 314 ein Selbstanzeigeformular generiert und ausgegeben. Dieses Selbstanzeigeformular beinhaltet vorzugsweise sämtliche relevanten Daten, wie zum Beispiel Angaben, die eine Identifizierung der Arztpraxis erlauben. In dem Schritt 316 wird das Selbstanzeigeformular zum Beispiel per E-Mail zurückgesendet. In dem Schritt 318 werden die eingehenden Selbstanzeigen gespeichert, womit die Patientendatenbanken, für die Teilnahme an der Datenerhebung festgelegt werden.

In dem Schritt 320 erfolgt dann die eigentliche Datenerhebung zum Beispiel für die Anwendungsbeobachtung; dies kann online oder auch offline erfolgen.

### Bezugszeichenliste

- Server Computer: 200
- Suchprofil: 202
- Mindestanzahl: 204
- Daten: 206
- Datenbank: 208
- Netzwerk: 210
- Server Computer: 212
- Server Computer: 214
- Datenbank: 216
- Programm: 218
- Adresse: 220
- Arbeitsplatzrechner: 222
- Datenbank: 224
- Programm: 226
- Adresse: 228
- Arbeitsplatzrechner: 230

## Patentansprüche

1. Computersystem mit mehreren verteilten Computersystemen und mit einem Zentral-Computersystem, wobei jedes der verteilten Computersysteme umfasst:
- eine Datenbank (216) zur Speicherung von Instanzen einer Entität,
- Mittel (212) zum Empfang eines Suchprofils (202) und einer Mindestanzahl (204),
- Mittel (218) zur Ermittlung einer Anzahl der Instanzen der Datenbank, für die das Suchprofil zutrifft,
- Mittel (218, 222) zur Ausgabe eines Signals, wenn die Anzahl die Mindestanzahl erreicht,
und das Zentral-Computersystem umfasst:
- Mittel (200) zum Empfang der Signale von den verteilten Computersystemen,
- Mittel (208) zur Speicherung von Adressen der verteilten Computersystemen, die ein Signal abgegeben haben,
- Mittel (202, 204) zur Speicherung des Suchprofils and der Mindestanzahl,
- Mittel (208) zur Übertragung des Suchprofils und der Mindestanzahl an die verteilten Computersysteme.

2. Computerprogrammprodukt, insbesondere digitales Speichermedium, mit Programmmitteln zur Abfrage von Daten aus einer Menge von unabhängigen Datenbanken zur Vorbereitung einer Datenerhebung, wobei jede der Datenbanken Instanzen einer Entität aufweist, zur Durchführung der folgenden Schritte:
- Übertragung eines Suchprofils und einer Mindestanzahl an zumindest eine Datenbank der Menge von unabhängigen Datenbanken,
- Ermittlung der Anzahl der Instanzen der Datenbank, für die das Suchprofil zutrifft,
- wenn die Anzahl die Mindestanzahl erreicht: Ausgabe eines Signals.

3. Verfahren zur Abfrage von Daten aus einer Menge von unabhängigen Datenbanken, wobei jede der Datenbanken Instanzen einer Entität aufweist, mit folgenden Schritten:
- Übertragung eines Suchprofils und einer Mindestanzahl an die Datenbanken der Menge von unabhängigen Datenbanken,
- für jede der Datenbanken:
a) Ermittlung der Anzahl der Instanzen der Datenbank, für die das Suchprofil zutrifft,
b) wenn die Anzahl die Mindestanzahl erreicht: Ausgabe eines Signals,
wobei die Ausgabe des Signals in Form einer Mitteilung für einen Benutzer der Datenbank erfolgt, und der Benutzer zu einer Aktion aufgefordert wird, beispielsweise zur Eingabe einer Bestätigung, zum Ausdrucken und Versenden eines Formulars oder zur Versendung eines Formulars auf elektronischem Wege, insbesondere als E-Mail.

4. Verfahren nach Anspruch 3, mit folgenden weiteren Schritten:
- Übertragung von Daten betreffend die Mitteilung an die Datenbanken,
- Zugriff auf eine Adresse einer Datenbank zur Erzeugung der Mitteilung,
- Sendung zumindest der Adresse nach einer Bestätigung der Mitteilung durch einen Benutzer,
- Speicherung von empfangenen Adressen.

5. Verfahren nach einem der vorhergehenden Ansprüche 3 oder 4, wobei die Übertragung online erfolgt oder zusammen mit einem Software-Update für ein Datenbankprogramm.

6. Verfahren nach einem der vorhergehenden Ansprüche 3, 4 oder 5, wobei es sich bei den Datenbanken um Patientendatenbanken von Arztpraxen oder anderen medizinischen Einrichtungen handelt und bei dem Suchprofil um eine Definition von in eine Anwendungsbeobachtung einzubeziehenden Patienten.

7. Verfahren nach Anspruch 6, wobei das Suchprofil basierend auf den Datenfeldern einer elektronischen Patientenkartei formuliert wird.

8. Computerprogrammprodukt, insbesondere digitales Speichermedium oder Datei, mit Programmmitteln zur Abfrage von Daten aus einer Menge von unabhängigen Datenbanken zur Vorbereitung einer Datenerhebung, wobei jede der Datenbanken Instanzen einer Entität aufweist, mit folgenden Schritten:
- Übertragung eines Suchprofils und einer Mindestanzahl an die Datenbanken der Menge von unabhängigen Datenbanken,
- für jede der Datenbanken:
a) Ermittlung der Anzahl der Instanzen der Datenbank, für die das Suchprofil zutrifft,
b) wenn die Anzahl die Mindestanzahl erreicht: Ausgabe eines Signals,
wobei die Ausgabe des Signals in Form einer Mitteilung für einen Benutzer der Datenbank erfolgt, und der Benutzer zu einer Aktion aufgefordert wird, beispielsweise zur Eingabe einer Bestätigung, zum Ausdrucken und Versenden eines Formulars oder zur Versendung eines Formulars auf elektronischem Wege, insbesondere als E-Mail.

9. Computerprogrammprodukt nach Anspruch 8, mit folgenden weiteren Schritten:
- Übertragung von Daten betreffend die Mitteilung an die Datenbanken,
- Zugriff auf eine Adresse einer Datenbank zur Erzeugung der Mitteilung,
- Sendung zumindest der Adresse nach einer Bestätigung der Mitteilung durch einen Benutzer,
- Speicherung von empfangenen Adressen.

10. Computerprogrammprodukt nach einem der vorhergehenden Ansprüche 8 oder 9, wobei die Übertragung online erfolgt oder zusammen mit einem Software-Update für ein Datenbankprogramm, und wobei es sich bei den Datenbanken um Patientendatenbanken von Arztpraxen oder anderen medizinischen Einrichtungen handelt und bei dem Suchprofil um eine Definition von in eine Anwendungsbeobachtung einzubeziehenden Patienten, und wobei das Suchprofil basierend auf den Datenfeldern einer elektronischen Patientenkartei formuliert wird.
